(19)

**Europäisches Patentamt**
**European Patent Office**
**Office européen des brevets**

(11) **EP 4 116 439 A1**

(12) **EUROPEAN PATENT APPLICATION**

(43) Date of publication:
**11.01.2023 Bulletin 2023/02**

(21) Application number: **21184246.3**

(22) Date of filing: **07.07.2021**

(51) International Patent Classification (IPC):
***C12Q 1/689*** *(2018.01)*

(52) Cooperative Patent Classification (CPC):
**C12Q 1/689**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Evonik Operations GmbH**
**45128 Essen (DE)**

(72) Inventors:
• **IGWE, Emeka Ignatius**
  **80333 München (DE)**
• **HUFNAGL, Marc**
  **28209 Bremen (DE)**
• **BÖHL, Florian**
  **60151 Neckargemünd (DE)**

• **KAPPEL, Andreas**
  **61479 Glashütten (DE)**
• **WEISSMANN, Michaela**
  **32130 Enger (DE)**
• **DARGATZ, Michelle**
  **33604 Bielefeld (DE)**
• **SMITH, Janet**
  **Mount Airy, GA 30563 (US)**

(74) Representative: **Evonik Patent Association**
**c/o Evonik Industries AG**
**IP Management**
**Bau 1042A/PB 15**
**Paul-Baumann-Straße 1**
**45772 Marl (DE)**

Remarks:
The complete document including Reference Table(s) and the Sequence Listing(s) can be downloaded from the EPO website

(54) **METHOD FOR MONITORING THE GUT MICROBIOME STATUS IN BROILER PRODUCTION**

(57)     The invention pertains to a method for controlling a process of broiler production, the method comprising monitoring the level of the *cpa* gene in pooled fecal sample material deriving from the broiler flock to be tested at consecutive points in time, wherein a reduction of the *cpa* level in the pooled fecal sample material over time is an indication of optimal broiler development within the production process with regard to their gut microbiome status.

EP 4 116 439 A1

**Description**

Field of the Invention

The present invention relates to a method for monitoring broiler development within a broiler production process with regard to their gut microbiome status.

Background of the invention

The intestinal microbiota members of the chicken are acquired vertically though the transmission of commensal microorganisms from mother to offspring via fecal deposits on eggs and naturally by direct contact during brooding [Stanley, et.al. (2013), "Highly variable microbiota development in the chicken gastrointestinal tract", PloS One, 8,12:1-7; Ding et. al. (2017), "Inheritance and Establishment of Gut Microbiota in Chickens", Front. Microbiol., 8, Article 1967]. This is followed by a continues enrichment of the gut with microbes from the immediate environment, feeds and from human activities during the growth cycle in the farms [Apajalahti et. al. (2004), "Characteristics of the gastrointestinal microbial communities, with special reference to the chicken. Worlds Poult. Sci. J. 60:223-232; Stanley et. al. (2013), "Highly variable microbiota development in the chicken gastrointestinal tract, PloS One, 8,12:1-7]. These different sources of starter cultures lead to the development of a highly diverse and relatively stable microbial community, consisting of mainly commensals and very low amounts of pathogens in healthy birds. When this ecosystem is in equilibrium, the host benefits with a profound impact on chickens' health and productivity [Maki, et. al. (2020), "Eggshell and environmental bacteria contribute to intestinal microbiota of growing chickens", Journal of Animal Science and Biotechnology, 11:60]. When there is a drastic change or imbalance in the composition of the microbiome, productivity is reduced because host vital energy required for tissue development is diverted to host-associated immune reactions aimed at eliminating pathogens and restoring equilibrium of the ecosystem.

It has been previously shown that after initial colonization of the gut of newly hatched birds, the species diversity and the complexity of the population structure of the microbiota increase with the bird's age until the microbiota maturations and stabilizes. This process is rapid, and it's completed within the first 3 weeks of the life of commercial chickens [Baldwin et. al. (2018), "At-hatch administration of probiotic to chickens can introduce beneficial changes in gut microbiota", PLoS ONE, 13; Lu et. al. (2003), "Diversity and succession of the intestinal bacterial community of the maturing broiler chicken", Appl. Environ. Microbiol., 69,11:6816-6824; Stanley et. al. (2013), "Highly variable microbiota development in the chicken gastrointestinal tract", PloS One, 8,12:1-7; Diaz Carrasco et. al. (2019), "Microorganisms 7:374]. Nonetheless, the duration of the development of the microbial community and the succession patterns of the intestinal microbiota species may vary, depending on factors like the genetic background, breeding conditions, and the farm management [Zhao et. al. (2013), "Quantitative genetic background of the host influences gut microbiomes in chickens", Sci. Rep., 3, 1163; Stanley et.al. (2014), "Microbiota of the chicken gastrointestinal tract: influence on health, productivity and disease", Appl. Microbiol. Biotechnol., 98, 4301-4310; Ding et. al. (2017), "Inheritance and Establishment of Gut Microbiota in Chickens", Front. Microbiol., 8, Article 1967; Pandit et. al. (2018), "Microbial diversity and community composition of caecal microbiota in commercial and indigenous Indian chickens determined using 16s rDNA amplicon sequencing, Microbiome, 6, 115; Zou et. al. (2018), "Lactobacillus elicits a 'Marmite effect' on the chicken cecal microbiome", npj Biofilms Microbiomes, 4, 27; Ngunjiri et. al. (2019), "Farm Stage, Bird Age, and Body Site Dominantly Affect the Quantity, Taxonomic Composition, and Dynamics of Respiratory and Gut Microbiota of Commercial Layer Chickens", Appl. Environ. Microbiol., 85, 18; Maki, et. al. (2020), "Eggshell and environmental bacteria contribute to intestinal microbiota of growing chickens", Journal of Animal Science and Biotechnology, 11:60].

*Clostridium perfringens* is a common pathogen present in chicken stables with known negative impact on the health and productivity of birds when they are disproportionately present in the gut microbiome. They are thought to originate from the hatchery and spreads through grow-out farms of an integrator [Craven et. al. (2001), "Incidence and tracking of Clostridium perfringens through an integrated broiler chicken operation", Avian Diseases", 47:707-711]. The mechanisms of colonization of the avian small intestinal tract by *Clostridium perfringens* are still not fully understood. Nonetheless, it is generally accepted that several factors like toxin production, and predisposing factors like coccidiosis causing damage to the gut mucosa, increased viscosity of intestinal contents due to high levels of non-starch polysaccharides in diet, and poor litter quality are essential for this process [Immerseel et.al. (2004), "Clostridium perfringens in poultry: an emerging threat for animal and public health", Avian Pathology, 33,6:537-549; Stanley et. al. (2014), "Microbiota of the chicken gastrointestinal tract: influence on health, productivity and disease", Appl. Microbiol. Biotechnol. 98:4301-4310; Moore et. al. (2016),"Necrotic enteritis predisposing factors in broiler chickens", Avian Pathol.,45, 275-281]. The proliferation of *Clostridium perfringens* in the gut is suggested to be favoured by antibiotics use and/or by other stressors that are capable of altering the normal microbiota (dysbacteriosis) of the bird [Latorre et. al. (2018), "Evaluation of the Epithelial Barrier Function and Ileal Microbiome in an Established Necrotic Enteritis Challenge Model in Broiler Chickens", Frontiers in Veterinary Science, 5, Article 199;Hawrelak et. al. (2004), "Cause of Intestinal Dysbiosis: A Review", Altern. Med. Rev. 9(2):180-197; Maslowski, K. M., Mackay, C. R. (2011),"Diet, gut microbiota and immune responses", Nature Immunology, 12:5 - 9]. Furthermore, the challenge of birds with pathogenic *C. perfringens* strains was shown to displace native *C. perfringens* from the GIT [Barbara et al. (2008)," Necrotic enteritis-producing strains of Clostridium perfringens displace non-necrotic enteritis strains from the gut of chicks", Vet. Microbiol. 126, 377-382; Timbermont et al. (2014)," Perfrin, a novel bacteriocin associated with netB positive Clostridium perfringens strains from broilers with necrotic enteritis", Vet. Res. 45, 40], causing a shift in the composition of gut microbiome in birds with physical signs of necrotic lesions as well as dysbiosis [Lacey et. al. (2018), "Clostridium perfringens-mediated necrotic enteritis is not influenced by the pre-existing microbiota but is promoted by large changes in the post-challenge", Veterinary Microbiology, 227,119-126]. It was also shown that the microbiome profile of birds that developed necrotic enteritis after a *Clostridium perfringens* challenge was different for that of healthy birds [Stanley et al.(2012), "Changes in the caecal microflora of chickens following Clostridium perfringens challenge to induce necrotic enteritis", Vet. Microbiol. 159, 155-162]. These findings suggest a link between gut microbiota composition and necrotic enteritis disease outcomes [Lin. et. al. (2017), "Disruption in the cecal microbiota of chickens challenged with Clostridium perfringens and other factors was alleviated by Bacillus licheniformis supplementation",PLoS One, 12; Xu et. al. (2018), "Bacillus licheniformis normalize the ileum microbiota of chickens infected with necrotic enteritis", Sci. Rep. 8, 1744].

As an animal pathogen, *C. perfringens* is responsible for several serious diseases including avian necrotic enteritis, which drains approximately US$ 6 billion/year from the global agricultural system [Wade, B., Keyburn, A.L. (2015), "The true cost of necrotic enteritis" World Poultry 31, 16-17]. *C. perfringens* is a Gram-positive, rod-shaped, spore forming, oxygen-tolerant anaerobe. Not all *C. perfringens* strains are virulent. The virulent *C. perfringens* strains are traditionally classified into five toxin types (A, B, C, D and E), based on the production of four suspected major toxins (alpha, beta, epsilon and iota). Depending on the toxins produced (major and additional toxins like NetB, Cpb2 and others), the *C. perfringens* sub-species specific syndromes/diseases can be induced in different host organisms [Rood, J. I. (1998) "Virulence genes of Clostridium perfringens"; Annual Review of Microbiology 52: 333-360]. The toxins are encoded by polynucleotide sequences located on the chromosome and/or on toxin plasmids [Popoff, M. R. and P. Bouvet (2013). "Genetic characteristics of toxigenic Clostridia and toxin gene evolution" Toxicon 75: 63-89].

*C. perfringens* causes mild to self-limiting infections in chickens, as well as necrotic enteritis (NE) which has both clinical and subclinical manifestations in poultry animals. Necrotic enteritis (NE) is an enteric disease of poultry that was first described in 1961. Early studies on NE suggested that the main virulence factor involved in the disease was the alpha-toxin (known as Cpa or Plc), which has phospholipase C and sphingomyelinase activity [Keyburn, A. L. et al. (2006) "Alpha-toxin of Clostridium perfringens is not an essential virulence factor in necrotic enteritis in chickens", Infection and Immunity 74(11): 6496-6500]. In more recent studies, the novel pore forming toxin, NetB, has been suggested to play a major key role in the development of this disease [Keyburn, A. L. et al. (2008) "NetB, a new toxin that is associated with avian necrotic enteritis caused by Clostridium perfringens" PLoS Pathogens 4(2)].

The clinical form of necrotic enteritis is characterised by an increase in mortality of birds, affecting up to 1% of bird in the flock each day and for several consecutive days during the last weeks of grow-out [Kaldhusdal & Løvland (2000), "The economical impact of Clostridium perfringens is greater than anticipated. World Poultry, 16, 50 - 51.]. In the subclinical forms of *Clostridium perfringens* infections, the birds might look healthy but there is considerable damage to the gut mucosa caused by the pathogen, which impairs the gut intestinal functional and leads to the production of birds with reduced weights and poor feed conversion ratios [Kaldhusdal & Løvland (2000), "The economical impact of Clostridium perfringens is greater than anticipated. World Poultry, 16, 50 - 51; Skinner et al. (2010), "An economic analysis of the impact of subclinical (mild) necrotic enteritis in broiler chickens", Avian Dis. 54, 4:1237-1240]. Despite the high levels of mortality caused by clinical forms of NE, subclinical form of NE is still considered to be of a more economic impact to the industry because it may remain undetected and untreated since there are no clinical manifestations (M'Sadeq et al. (2015), "Towards the control of necrotic enteritis in broiler chickens with in-feed antibiotics phasing-out Worldwide", Animal Nutrition 1:11; Dahiya et al.(2006)," MD. Potential strategies for controlling necrotic enteritis in broiler chickens in post-antibiotic era". Anim. Feed Sci. Technol., 129:60].

The gut microbiota is characterized by targeted amplification of the small subunits of the 16S ribosomal gene for bacteria and Archaea, the 18S rRNA gene for eukaryotic species and the nuclear ribosomal internal transcribed spacer (ITS) regions for Fungi [Cressman et. al. (2010)," Interrelations between the microbiotas in the litter and in the intestines of commercial broiler chickens", Appl. Environ. Microbiol., 76, 19:6572-82]; this is followed by a shotgun sequencing of the amplicons and a bioinformatics analysis of the sequence data to estimate the relative abundance of sequences in the samples, and assign specific taxonomic units to them [Borda-Monila et. al. (2018), "Current Perspectives of the Chicken Gastrointestinal Tract and Its Microbiome", Computational and Structural Biotechnology Journal ,16, 131-139]. Most available studies on chicken gut microbiota are based on sequencing the hyper variable region of the 16S rRNA gene [Danzeisen et.al. (2011), "Modulations of the chicken cecal microbiome and metagenome in response to anticoccidial and growth promoter treatment", PLoS ONE, 6:11; Yilmaz et. al. (2014), "Uniting the classification of cultured and uncultured bacteria and archaea using 16S rRNA gene sequences", Nat. Rev. Microbiol., 12,9:635-45; Stanley et. al. (2013), "Highly variable microbiota development in the chicken gastrointestinal tract", PLoS ONE, 8:12].

The characterisation of the gut microbiome by ribosomal gene sequencing has been used successfully in research settings, however, its application in commercial poultry production is limited by the complexity of its protocols, a long time-to-result, high costs, and the reliance on a strong bioinformatics competence in order to be able draw any meaningful conclusion from results obtained. Microbial profiles generated by sequencing are highly influenced by the study design, sequencing platform and chemistry used, making the comparison of data across studies very difficult [Sergeant, et. al. (2014), "Extensive microbial and functional diversity within the chicken cecal microbiome", PLoS ONE,14, 9:3; Borda-Molina et. al. (2016)," Insights into broilers' gut microbiota fed with phosphorus, calcium and phytase supplemented diets", Front Microbiol. 7; Stanley et. al. (2013), "Highly variable microbiota development in the chicken gastrointestinal tract. PLoS ONE, ,8:12]. Moreover, since the lifespan of commercial broiler are short (few weeks), there is still a need for the development of rapid and quicker methods of assessing the gut microbiome, which will allow broiler producers enough time to intervene before grow-out of the flock in question.

In view of the above it was the objective of the present invention to provide new and alternative methods for monitoring broiler development within a broiler production process with regard to their gut microbiome status.

Summary of the invention

The inventors have unexpectedly found that a reduction of the *cpa* level in the pooled fecal sample material deriving from a broiler flock over time is an indication of optimal broiler development within the production process with regard to their gut microbiome status.

As used in the context of the present invention, the term "gut microbiome status" refers to the increase of pathogens like *C. perfringens* with the age of the chickens as a result of a shift in microbiome composition in gut induced by stressors (also designated as "bad" microbiome status), and the decrease in levels of *C. perfringens* with the age of the chickens during developments (also designated as "good" microbiome status.

In other words, when monitoring the level of the *cpa* gene in pooled fecal sample material deriving from the broiler flock to be tested at consecutive points in time, an optimal process is characterized by the reduction of *cpa* over time (i.e. with age); and a sub-optimal process is characterized by a continuous increase of cpa level over time (i.e. with age), and in particular in an increase over a threshold of 5 log(cpa copies)/gram feces.

Accordingly, the present invention provides a method for controlling a process of broiler production, the method comprising monitoring the level of the *cpa* gene in pooled fecal sample material deriving from the broiler flock to be tested at consecutive points in time, wherein a reduction of the *cpa* level in the pooled fecal sample material over time is an indication that the broiler flock is developing a normal/balanced gut microbiome (i.e. that the composition of the gut microbiome is normal).

Detailed description of the invention

The nucleotide sequence of *cpa* is known in the art. However, for the sake of completeness, the consensus sequence of *cpa* is indicated in SEQ ID NO: 1.

A "pooled fecal sample" is to be understood as a composite sample from randomly selected separate fecal samples, one sample taken with one or several moistened fabric swabs or pooled samples made up of separate samples of fresh samples taken at random from a number of sites in the house or space in which the avian population is kept.

Suitable sample volumes are, for example, 0.1 to 20 ml, in particular 0.2 to 10 ml, preferably 0.5 to 5 ml. Suitable sample masses are, for example 0.1 to 20 g, in particular 0.2 to 10 g, preferably 0.5 to 5 g.

The sample size (i.e. the number of fecal samples to be taken; each sample taken at a specific site within the animal house) has to be determined in view of the actual stocking density, i.e. with the actual number of animals belonging to the avian population to be tested.

The sample size may be calculated using the following formula:

$$n_0 = \frac{Z^2 pq}{e^2}$$

wherein

no is the sample size recommendation
Z is 1.96 for 95% confidence level
p is the estimated portion of the population with the attribute in question q is 1-p, and
e is the confidence interval expressed as decimal.

In general, a minimum of 80 to 100 individual fecal samples are sufficient for most livestock avian populations. Broilers are usually kept in flocks which can consist of > 20000 birds in one house.

As an example, for a broiler flock of 20000 animals, 96 individual samples are required for a confidence level of 95%.

The above formula is particularly suitable for determining the sample size required for large population.

For smaller populations, (<= 100), the sample size recommendation no as obtained with the above formula may be further adjusted in accordance with the following formula:

$$n = \frac{n_0}{1 + \frac{(n_0 - 1)}{N}}$$

wherein

N is the population size, and
n is the adjusted sample size.

For obtaining the pooled fecal sample material as required in step (a), several sampling methods may be used.

In one embodiment, the pooled fecal sample may be obtained by systematic grid sampling (systematic random sampling). For this method, the area in which the avian population is kept is divided in a grid pattern of uniform cells or sub-areas based on the desired number of individual fecal samples (i.e. the sample size). Then, a random sample collection site is identified within the first grid cell and a first sample is taken at said site. Finally, further samples are obtained from adjacent cells sequentially - e.g. in a serpentine, angular or zig-zag fashion - using the same relative location within each cell. A random starting point can be obtained with a dice or a random number generator.

The above process may optionally be repeated for replicate samples. That is, a new random position is established for the single collection point to be repeated in all of the cells. By analyzing replicate samples, variabilities in the estimate of the mean provided by the original samples may be determined.

The sample size corresponds to the number of cells in the grid pattern in case one sample is to be taken per cell. In general, in case x samples are to be taken per cell, the sample size is the number of cells, divided by x.

The systematic grid sampling method can be easily implemented in the field. Thereby, over- or underrepresentation of subareas can be avoided.

Another sampling method is stratified random sampling (i.e. random sampling within a grid). Herein, samples are obtained sequentially from adjacent grid cells, but the location of the sample within each cell is random.

Alternatively, the samples may be taken by simple random sampling, where the samples are taken from random locations (without gridding) across the area in which the animals are kept. For this method, a formal approach for determining the random sample locations must be used, e.g. based upon a random number generator.

The samples may be collected manually with a spatula or a similar device and are immediately transferred into a sample collection vessel or tube.

In an alternative embodiment, the pooled fecal sample may be obtained using the overshoe method while walking through the house using a route that will produce representative samples for all parts of the house or the respective sector. Such route may e.g. be uniformly shaped serpentines or sinuous lines, angular lines or zigzag lines. Boot swabs being sufficiently absorptive to soak up moisture are particularly suitable. However, tube gauze socks are also acceptable.

For the monitoring approach according to the present invention, the fecal samples are generally to be collected and analyzed on a weekly, daily or hourly basis. In a preferred embodiment, the fecal samples are collected at consecutive days. Preferably, sample collection and sample analysis are started before day 14. For example, sample collection and sample analysis are started from day 1, from day 5, from day 10 or from day 13 on a daily basis.

For broiler flocks, fecal samples may preferably be collected and analyzed on a daily basis during the initial growth phase (starter phase, day 5 to day 10), and/or during the enhanced growth phase (day 11 to day 18) and, optionally, also on a later stage.

In one embodiment, the fecal sample material from the broiler flock is collected and analyzed on a daily basis starting from day 10.

The *cpa* gene may be isolated from the fecal sample material, prior to quantification. Polynucleotide isolation can for example, be performed via extraction using the cetyltrimethylammoniumbromid (CTAB) method or by diverse commercial nucleic acid extraction kits, in which cell lysis is achieved either through chemical lysis and/or by mechanical cell disruption and nucleic acid is captured on silica matrices or on silica-cladded magnetic beads. Commercial extraction kits specialized on fecal material or harsh material are particularly suitable.

The *cpa* marker gene may be detected and/or quantified by commonly known methods such as sequencing, hybridization or various PCR techniques known in the art.

In an alternative embodiment, the *cpa* marker gene contained in the animal sample can be quantified directly, for example via PCR, qPCR, sequencing or hybridization techniques.

The present invention provides the above-described non-invasive methods to monitor gut microbiome status which can be performed *ante mortem.* This enables the farmer to take measures against the C. *perfringens* contamination at an early stage.

Accordingly, the present invention also pertains to the use of the aforementioned method for determining the necessity of nutritional or therapeutic interventions. More specifically, in case the *cpa* level in the pooled fecal sample material does not decrease over time, nutritional or therapeutic interventions may be appropriate.

Such interventions or measures include feeding or administering health-promoting substances, such as zootechnical feed additives, or therapeutic agents. The term "administering" or related terms includes oral administration. Oral administration may be via drinking water, oral gavage, aerosol spray or animal feed. The term "zootechnical feed additive" refers to any additive used to affect favorably the performance of animals in good health or used to affect favorably the environment. Examples for zootechnical feed additives are digestibility enhancers, i.e. substances which, when fed to animals, increase the digestibility of the diet, through action on target feed materials; gut flora stabilizers; micro-organisms or other chemically defined substances, which, when fed to animals, have a positive effect on the gut flora; or substances which favorably affect the environment. Preferably, the health-promoting substances are selected from the group consisting of probiotic agents, prebiotic agents, botanicals, organic/fatty acids, bacteriophages and bacteriolytic enzymes or any combinations thereof.

Applications of the methods according to the invention are for example (i) aiding in the diagnosis and/or prognosis of infections/contaminations with *C. perfringens* strains; (ii) monitoring the progress and/or reoccurrence of this condition, or (iii) aiding in the evaluation of treatment efficiency for an animal population undergoing or contemplating treatment. Applications of the invention in particular help to avoid loss in animal performance like weight gain and feed conversion.

The methods according to the present invention are particularly suitable for use in antibiotic-free broiler production. As an alternative to collecting and pooling fecal samples within the broiler house, the methods according to the present invention may also be used for wastewater-analyses.

In the following, the invention is illustrated by non-limiting examples and exemplifying embodiments.

Brief description of the Figures

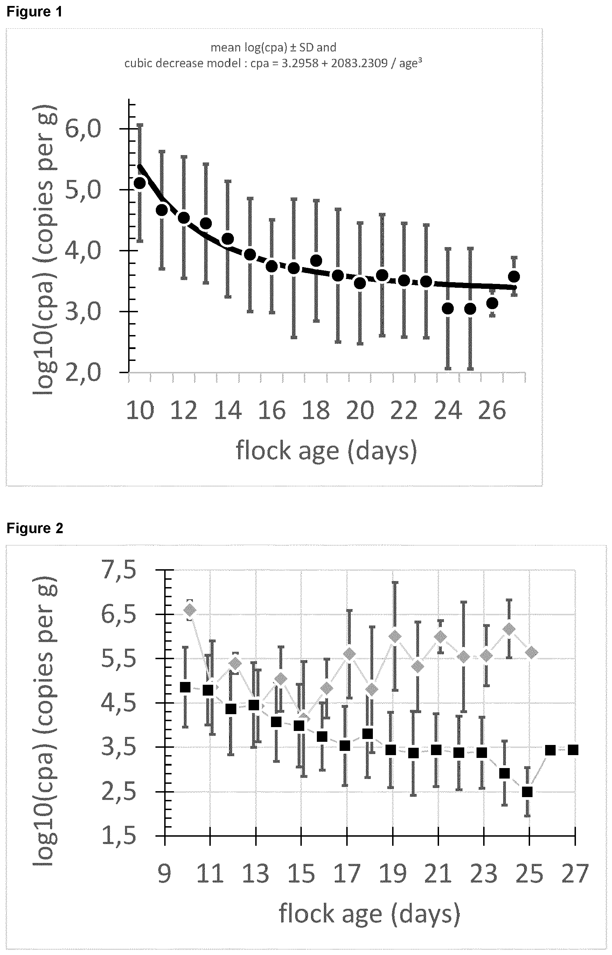

Figure 1 shows the decrease of *cpa* with broiler age during live production (optimal process).
Figure 2 shows the increase in the levels of *cpa* with age during live production as indication of C. *perfringens* contamination; cpa levels were significantly different and consistently higher form day 17 onwards when compared to the optimal process

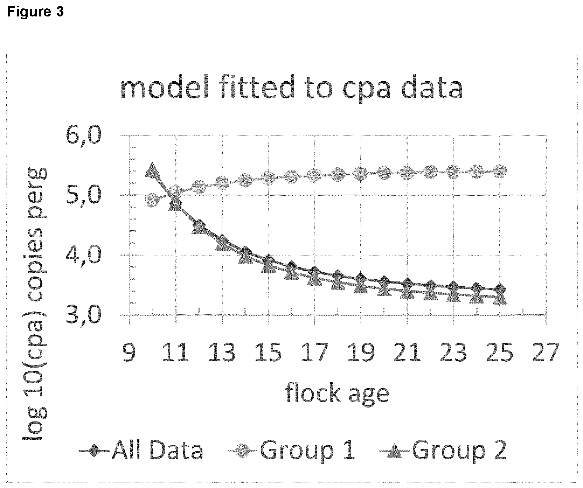

Figure 3 shows the relationship between *cpa* and age in a cubic regression fit model: cpa levels eroded with increasing age in flocks with normal/balanced gut microbiome development but increased with age in flocks with imbalanced gut microbiome development.

Examples

*C. perfringens* (CP) was used as pathogen to be detected in the avian population because their growth is favored by any change that could cause an imbalance in gut microbiome; thus, the dynamic of C.P during broiler production/development is therefore a reliable indicator of the gut microbiome status. Herein, a well-conserved and chromosomally located gene (cpa) target of *Clostridium perfringens* was used in assessing its dynamics during broiler production.

About 20,000 broilers were randomly assigned to broiler houses as part of the normal chicken placement procedures of the company, in accordance to the American Humane Association certified program, which limits density to 6.2 pounds /square foot at slaughter, including substantial management, and auditing needs. All flocks were managed according to company's standard protocols, which are in line with breeder's recommendations for lighting, temperature, and ventilation. Feeds consisted of basal diet (corn and soy) adjusted for birds' requirements for starter, grower & finisher feeds. General flock conditions were monitored daily: the availability of feed and water, temperature control, and any unusual conditions. Dead birds were removed and necropsied to determine cause of death and debilitated birds were culled to avoid further suffering.

Sample collection

Fecal samples and flock performance data from several standard broiler live production processes were collected daily from days 10 to 28 from 139 flocks. Furthermore, flocks were monitored daily for signs of enteric diseases such as bacterial dysbacteriosis and/or NE outbreak (increase daily mortality rate at the expected NE occurrence window (11-28 days) and typical NE lesion as well as with necropsy of dead birds for typical NE lesions.

At each collection time point or event, 24 individual samples were picked up from each quadrant of the house with a plastic tong, walking each quadrant in a zig-zag fashion. To avoid cross contamination of samples, new sterile tong was used for each house as well as prescribed biosecurity measures were observed. Furthermore, debris such as wood shavings, litter, etc., were removed from the samples before all samples from the 4 quadrants were composited to form a single pooled sample (consisting of 96 individual fecal samples) in a sterile sample collection bag. The samples were placed on ice and transferred to the laboratory for storage at -80° C.

DNA extraction

Each bag with the pooled 96 samples was allowed to thaw slowly at room temperature; then, the feces were transferred into a sterile container and mixed thoroughly with a sterile tongue depressor. Five (5) grams of the homogenized sample were transferred to a proprietary sample collection tube, containing 20 ml of stabilization buffer and glass beads. Fecal samples in the sample collection tubes are stable for up to 7 days at +15°C to + 30°C.

The tube containing the fecal sample was incubated at 70°C for 20 minutes in a water bath. The tube was then transferred to a Poly Mix Mill (bead beater) for homogenization at 20 Hz for 15 minutes. At the end of the homogenization, the sample was centrifuged at 2000g for 5 minutes, and 500 $\mu$l of the supernatant was used for DNA extraction. DNA extraction was performed with the King Fisher Flex system (Thermo Fisher, USA), adhering to the protocol of Evonik's proprietary fecal extraction kit.

The King Fisher instrument was prepared by uploading a predefined program ("Cper_Extraction_01") defining the various steps of the extraction process; sampling tips, DNA elution plate, wash plates and sample plate were prepared as described below.

A 96 tips comb was inserted in an empty deep well plate and placed it in the instrument. This was followed by the introduction of 100$\mu$l of the elute buffer in an elution plate and this plate was also placed in the instrument. Furthermore, 500 $\mu$l of wash buffers 3, 2 and 1 where place in each well of 3 different wash plates respectively, and these plates were placed on the instrument in the same order. Finally, 300 $\mu$l of lysis buffer, 25 $\mu$l magnetic beads, 20 $\mu$l enhancer, 10 $\mu$l internal control and 500 $\mu$l of the supernatant from the fecal sample were added to each well of a sample plate. After placing the sample plate on the instrument, the extraction was started by pressing the start button.

DNA Quantification

For the quantification of *Clostridium perfringens* marker (cpa target gene) in the DNA extracted from the pooled fecal samples, ScreenFloX PCR C. perfringens kit (Evonik Nutrition & Care GmbH, Germany) was used. In brief, 20 $\mu$l master mix consisting of 5 $\mu$l Master A, 15 $\mu$l master B and 1 $\mu$l of IC (internal control) was prepared according to the instruction of the manufacturer. Enough master mix was prepared to accommodate the running of all samples, non-template controls (NTC) and 4 standards (S1 to S4) in duplicates. 20 $\mu$l of the master mix were dispensed into individual wells of a 96 well plate. Then, a 10 $\mu$l of the extracted DNA sample was transferred into each well. 10 $\mu$l of the respective standard and 1 $\mu$l of IC were transferred to each standard well accordingly. To prepare a NTC, 10 $\mu$l of sterile nuclease free water and 1 $\mu$l of IC were transferred to the NTC wells each. The contents of the plate were mixed thoroughly with a multi-channel pipet, and the plate was sealed with a Clear Weld Seal Mark II foil. film. The plate was centrifuged for 30 seconds at 1000 g (~3000 rpm). Finally, the plate was run on a CFX96 real time PCR instrument (Bio Rad, Germany) with the following PCR conditions: 45 cycles of denaturation at 95°C for 15 seconds, annealing at 58°C for 45 seconds and extension at 72°C for 15 seconds. Data were acquired during the amplification phase of the QPCR run. At the end of the run, data received from the BioRad CFX96 were preprocessed with the Bio- Rad CFX Manager 3.1 and exported to Excel 2013 for further analysis. The quantification of markers in samples were determined from the standard curve constructed with standard solutions (S1 to S4) containing equal concentrations of both targets. The concentrations of *netB* in S1, S2, S3 and S4 are $10^4$ copies/$\mu$l, $10^3$ copies/$\mu$l, $10^2$ copies/$\mu$l and $10^1$ copies/$\mu$l respectively. The log of the standards were plotted along the x-axis, while the Ct (cycle thresholds) were plotted along the y-axis. The resulting linear regression line [y=mx + b or Ct= m (log quantity) + b] was used to determine the concentrations of the targets in the sample tested.

List of primers and probe used for the qPCR to quantify levels of expression of *cpa*:

| Target | Primers and Probes (where applicable) | Probe reporter |
|---|---|---|
| *cpa* | Forward: 5'- TACATATCAACTAGTGGTGA -3' (SEQ ID NO.: 2) | |
| | Reverse: 5'- ATTCTTGAGTTTTTCCATCC -3' (SEQ ID NO.: 3) | |
| | Probe: 5'- TGGAACAGATGACTACATGTATTTTGG-3 (SEQ ID NO.: 4) | Cy5 |

Statistical methods

Statistical analyses were performed using the software R version 3.5.1. All available data from 139 flocks were categorized into three different groups. Group 1 were flocks confirmed for enteric diseases like NE or dysbacteriosis and with imbalanced gut microbiome. Flocks were confirmed with classical method (necropsy of birds) by a veterinarian. Group 2 were flocks with no level of *netB* detected at any time point of the flocks' grow out; these flocks were not confirmed for any enteric disease like NE or dysbacteriosis, so with a balanced/normal gut microbiome. The third group contained flocks where *netB* was measured at least at one-time point of sampling, but also these flocks were not confirmed by a veterinarian until grow out for any enteric disease like NE or dysbacteriosis. For each group all data were aggregated by flock age. The data were tested for a normal distribution using the Shapiro test and a p value threshold of 0.05. Equal distribution of variances was tested using the Levene test again with a p-value threshold of 0.05. Both conditions are requirements for calculating an ANOVA to identify whether the *cpa* values measured at a certain day differ significantly between the "Group 1" and "Group 2". Differences between certain days were determined using the Tukey post-hoc test. As for day 25 no normal distribution of the data was determined based on the Shapiro Test (but on Kolmogorov Smirnov Test) additionally the parametric Kruskall Wallis test was calculated. The p-values displayed in the table and based on the ANOVA and the Kruskall-Wallis (KW) test were obtained. In this case the ANOVA p-values are the more robust ones and should be taken into consideration here.

The decrease in cpa with age was determined by fitting a cubic function of the form:

$$log_{10}(cpa) = a + \frac{b}{flock\ age^3}$$

to the data by using the R package nls. Again, the two different groups "Group1" and "Group2" were used, but additionally the model was fitted to all available data. The parameter a determines the offset. If the flock age is infinite the second term $b/(flock\ age)^3$ is zero and therefore "a" can be considered as the base level of cpa. The parameter "b" determines the start level and partly the decrease/decay rate. If "b" is zero (or not significantly different from 0) there is no decay as the second term is zero. For any age the cpa level is then equal parameter "a".

The following values were obtained:

| | a | std error | p-value | B | std error | p-value | deg of freedom |
|---|---|---|---|---|---|---|---|
| All data | 3.2958 | 0.04758 | <2e-16 | 2083.2309 | 135 | <2e-16 | 1123 |
| *Group 1* | 5.4304 | 0.2351 | <2e-16 | -516.9537 | 604.7523 | 0.396 | 60 |
| Group 2 | 3.156 | 0.05605 | <2e-16 | 2269 | 160.9 | <2e-16 | 688 |

For all models the parameter "a" was significantly different from 0 and for the Group 1NE data, it was higher than for the Group 2 or all data (Group 1, 2 &3). Parameter "b" was not significantly different from zero for the NE data. Here also the parameters estimate + the standard error included the zero. This means that - based on this chosen model - it cannot be stated that there was a decrease of cpa with age for group 1 flocks where enteric disease like NE or dysbacteriosis was confirmed by the veterinarian , while there was a decrease of cpa with age for the group 2 flocks, where enteric disease like NE or dysbacteriosis was not confirmed by veterinarian through grow out.

**Table 1**

| | "Group 1 | "Group 2 | | | | |
|---|---|---|---|---|---|---|
| Age | Mean cpa | mean cpa | p-value KW | | p-value ANOVA | |
| 10 | 6,60 | 4,85 | 0.0285 | * | 0.7946 | n.sig.diff |
| 11 | 4,85 | 4,79 | 0.4694 | n.sig.diff | 10.000 | n.sig.diff |

(continued)

|  | "Group 1 | "Group 2 |  |  |  |  |
|---|---|---|---|---|---|---|
| Age | Mean cpa | mean cpa | p-value KW |  | p-value ANOVA |  |
| 12 | 5,39 | 4,37 | 0.0188 | * | 0.9564 | n.sig.diff |
| 13 | 4,43 | 4,45 | 0.9088 | n.sig.diff | 10.000 | n.sig.diff |
| 14 | 5,04 | 4,07 | 0.0297 | * | 0.9149 | n.sig.diff |
| 15 | 4,14 | 3,98 | 0.5568 | n.sig.diff | 10.000 | n.sig.diff |
| 16 | 4,83 | 3,74 | 0.1019 | n.sig.diff | 0.9995 | n.sig.diff |
| 17 | 5,60 | 3,54 | 0.0003 | *** | 0.0000 | *** |
| 18 | 4,80 | 3,80 | 0.0679 | * | 0.8956 | n.sig.diff |
| 19 | 6,00 | 3,44 | 0.0024 | ** | 0.0000 | *** |
| 20 | 5,32 | 3,37 | 0.0020 | ** | 0.0019 | ** |
| 21 | 6,00 | 3,44 | 0.0002 | *** | 0.0000 | *** |
| 22 | 5,54 | 3,37 | 0.0083 | ** | 0.0211 | * |
| 23 | 5,57 | 3,38 | 0.0263 | * | 0.2222 | n.sig.diff |
| 24 | 6,17 | 2,92 | 0.0187 | * | 0.0004 | *** |
| 25 | 5,64 | 2,50 | 0.1069 | n.sig.diff | 0.1959 | n.sig.diff |

**Table 2**

| Flock age | All | Group1 | Group 2 |
|---|---|---|---|
| 10 | 5,3790309 | 4,9134463 | 5,425 |
| 11 | 4,86096221 | 5,04200503 | 4,86073328 |
| 12 | 4,50137344 | 5,13123698 | 4,4690787 |
| 13 | 4,24401616 | 5,19510018 | 4,18877196 |
| 14 | 4,05499493 | 5,24200579 | 3,98289504 |
| 15 | 3,9130536 | 5,27722853 | 3,8282963 |
| 16 | 3,80440129 | 5,3041906 | 3,70995508 |
| 17 | 3,7198242 | 5,3251784 | 3,61783595 |
| 18 | 3,65300694 | 5,3417591 | 3,54506036 |
| 19 | 3,59952225 | 5,35503133 | 3,48680624 |
| 20 | 3,55620386 | 5,36578079 | 3,439625 |
| 21 | 3,52074665 | 5,37457949 | 3,40100594 |
| 22 | 3,49144528 | 5,38185063 | 3,36909166 |
| 23 | 3,46701977 | 5,38791182 | 3,34248804 |
| 24 | 3,44649668 | 5,39300462 | 3,32013484 |
| 25 | 3,42912678 | 5,39731496 | 3,301216 |

## Claims

1. A method for controlling a process of broiler production,

the method comprising monitoring the level of the *cpa* gene in pooled fecal sample material deriving from the broiler flock to be tested at consecutive points in time,

wherein a reduction of the *cpa* level in the pooled fecal sample material over time is an indication of optimal broiler development within the production process with regard to their gut microbiome status.

2. The method according to claim 1, wherein the fecal sample material is collected and analyzed on a weekly, daily or hourly basis.

3. The method according to any one of the preceding claims, wherein the fecal sample material is collected and analyzed on a daily basis starting from day 10.

4. The method according to any one of the preceding claims, wherein the sample masses is between 0.2 and 10 g, preferably between 0.5 and 5 g.

5. The method according to any one of the preceding claims, wherein the sample size is calculated using the following formula:

$$n_0 = \frac{Z^2 pq}{e^2}$$

wherein

no is the sample size recommendation
Z is 1.96 for 95% confidence level
p is the estimated portion of the population with the attribute in question q is 1-p, and
e is the confidence interval expressed as decimal.

6. The method according to any one of the preceding claims, wherein the *cpa* marker gene contained in the pooled fecal sample is quantified via PCR, preferably via qPCR.

**Figure 1**

mean log(cpa) ± SD and
cubic decrease model : cpa = 3.2958 + 2083.2309 / age³

**Figure 2**

**Figure 3**

## EUROPEAN SEARCH REPORT

Europäisches
Patentamt

European
Patent Office

Office européen
des brevets

| Application Number |
| --- |
| EP 21 18 4246 |

### DOCUMENTS CONSIDERED TO BE RELEVANT

| Category | Citation of document with indication, where appropriate, of relevant passages | Relevant to claim | CLASSIFICATION OF THE APPLICATION (IPC) |
| --- | --- | --- | --- |
| X | WO 2019/238561 A1 (EVONIK OPERATIONS GMBH [DE]) 19 December 2019 (2019-12-19) * the whole document * | 1-5 | INV. C12Q1/689 |
| A | GURJAR ET AL: "Real-time multiplex PCR assay for rapid detection and toxintyping of Clostridium perfringens toxin producing strains in feces of dairy cattle", MOLECULAR AND CELLULAR PROBES, ACADEMIC PRESS, LONDON, GB, vol. 22, no. 2, 6 March 2008 (2008-03-06), pages 90-95, XP022513580, ISSN: 0890-8508, DOI: 10.1016/J.MCP.2007.08.001 | 1-5 | |
| A | STANLEY DRAGANA ET AL: "Microbiota of the chicken gastrointestinal tract: influence on health, productivity and disease", APPLIED MICROBIOLOGY AND BIOTECHNOLOGY, SPRINGER BERLIN HEIDELBERG, BERLIN/HEIDELBERG, vol. 98, no. 10, 19 March 2014 (2014-03-19), pages 4301-4310, XP035318179, ISSN: 0175-7598, DOI: 10.1007/S00253-014-5646-2 [retrieved on 2014-03-19] | 1-5 | |

-/--

| | TECHNICAL FIELDS SEARCHED (IPC) |
| --- | --- |
| | C12Q |

The present search report has been drawn up for all claims

| Place of search | Date of completion of the search | Examiner |
| --- | --- | --- |
| The Hague | 14 December 2021 | Cornelis, Karen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 21 18 4246

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | YUANYUAN WU ET AL: "Effects of supplementation on the growth performance and gut health of broiler chickens with -induced necrotic enteritis", JOURNAL OF ANIMAL SCIENCE AND BIOTECHNOLOGY, BIOMED CENTRAL LTD, LONDON, UK, vol. 9, no. 1, 25 January 2018 (2018-01-25), pages 1-14, XP021252922, DOI: 10.1186/S40104-017-0220-2 | 1-5 | |
| X | GAUCHER MARIE-LOU ET AL: "Recurring Necrotic Enteritis Outbreaks in Commercial Broiler Chicken Flocks Strongly Influence Toxin Gene Carriage and Species Richness in the Resident Clostridium perfringens Population", FRONTIERS IN MICROBIOLOGY, vol. 8, 17 May 2017 (2017-05-17), XP55871095, Lausanne ISSN: 1664-302X, DOI: 10.3389/fmicb.2017.00881 * the whole document * | 1-5 | |
| A | SHAN WEI ET AL: "Abundance of pathogens in the gut and litter of broiler chickens as affected by bacitracin and litter management", VETERINARY MICROBIOLOGY, vol. 166, no. 3-4, 25 October 2013 (2013-10-25), pages 595-601, XP055672930, DOI: 10.1016/j.vetmic.2013.06.006 | 1-5 | |

TECHNICAL FIELDS SEARCHED (IPC)

The present search report has been drawn up for all claims

| Place of search | Date of completion of the search | Examiner |
|---|---|---|
| The Hague | 14 December 2021 | Cornelis, Karen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 18 4246

This annex lists the patent family members relating to the patent documents cited in the above-mentioned European search report.
The members are as contained in the European Patent Office EDP file on
The European Patent Office is in no way liable for these particulars which are merely given for the purpose of information.

14-12-2021

| Patent document cited in search report | Publication date | Patent family member(s) | Publication date |
|---|---|---|---|
| WO 2019238561 A1 | 19-12-2019 | BR 112020025403 A2 | 16-03-2021 |
| | | CA 3103172 A1 | 19-12-2019 |
| | | CN 112236533 A | 15-01-2021 |
| | | EP 3807421 A1 | 21-04-2021 |
| | | KR 20210021014 A | 24-02-2021 |
| | | US 2021262031 A1 | 26-08-2021 |
| | | WO 2019238561 A1 | 19-12-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **STANLEY.** Highly variable microbiota development in the chicken gastrointestinal tract. *PloS One,* 2013, vol. 8 (12), 1-7
- **DING.** Inheritance and Establishment of Gut Microbiota in Chickens. *Front. Microbiol.,* 2017, vol. 8
- **APAJALAHTI.** Characteristics of the gastrointestinal microbial communities, with special reference to the chicken. *Worlds Poult. Sci. J.,* 2004, vol. 60, 223-232
- **MAKI.** Eggshell and environmental bacteria contribute to intestinal microbiota of growing chickens. *Journal of Animal Science and Biotechnology,* 2020, vol. 11, 60
- **BALDWIN.** At-hatch administration of probiotic to chickens can introduce beneficial changes in gut microbiota. *PLoS ONE,* 2018, vol. 13
- **LU.** Diversity and succession of the intestinal bacterial community of the maturing broiler chicken. *Appl. Environ. Microbiol.,* 2003, vol. 69 (11), 6816-6824
- **DIAZ CARRASCO.** *Microorganisms,* 2019, vol. 7, 374
- **ZHAO.** Quantitative genetic background of the host influences gut microbiomes in chickens. *Sci. Rep.,* 2013, vol. 3, 1163
- **STANLEY.** Microbiota of the chicken gastrointestinal tract: influence on health, productivity and disease. *Appl. Microbiol. Biotechnol.,* 2014, vol. 98, 4301-4310
- **PANDIT.** Microbial diversity and community composition of caecal microbiota in commercial and indigenous Indian chickens determined using 16s rDNA amplicon sequencing. *Microbiome,* 2018, vol. 6, 115
- **ZOU.** Lactobacillus elicits a 'Marmite effect' on the chicken cecal microbiome. *Biofilms Microbiomes,* 2018, vol. 4, 27
- **NGUNJIRI.** Farm Stage, Bird Age, and Body Site Dominantly Affect the Quantity, Taxonomic Composition, and Dynamics of Respiratory and Gut Microbiota of Commercial Layer Chickens. *Appl. Environ. Microbiol.,* 2019, vol. 85, 18
- **CRAVEN.** Incidence and tracking of Clostridium perfringens through an integrated broiler chicken operation. *Avian Diseases,* 2001, vol. 47, 707-711
- **IMMERSEEL.** Clostridium perfringens in poultry: an emerging threat for animal and public health. *Avian Pathology,* 2004, vol. 33 (6), 537-549

- **MOORE.** Necrotic enteritis predisposing factors in broiler chickens. *Avian Pathol.,* 2016, vol. 45, 275-281
- **LATORRE.** Evaluation of the Epithelial Barrier Function and Ileal Microbiome in an Established Necrotic Enteritis Challenge Model in Broiler Chickens. *Frontiers in Veterinary Science,* 2018, vol. 5
- **HAWRELAK.** Cause of Intestinal Dysbiosis: A Review. *Altern. Med. Rev.,* 2004, vol. 9 (2), 180-197
- **MASLOWSKI, K. M. ; MACKAY, C. R.** Diet, gut microbiota and immune responses. *Nature Immunology,* 2011, vol. 12, 5-9
- **BARBARA et al.** Necrotic enteritis-producing strains of Clostridium perfringens displace non-necrotic enteritis strains from the gut of chicks. *Vet. Microbiol.,* 2008, vol. 126, 377-382
- **TIMBERMONT et al.** Perfrin, a novel bacteriocin associated with netB positive Clostridium perfringens strains from broilers with necrotic enteritis. *Vet. Res.,* 2014, vol. 45, 40
- **LACEY.** Clostridium perfringens-mediated necrotic enteritis is not influenced by the pre-existing microbiota but is promoted by large changes in the post-challenge. *Veterinary Microbiology,* 2018, vol. 227, 119-126
- **STANLEY et al.** Changes in the caecal microflora of chickens following Clostridium perfringens challenge to induce necrotic enteritis. *Vet. Microbiol.,* 2012, vol. 159, 155-162
- **LIN.** Disruption in the cecal microbiota of chickens challenged with Clostridium perfringens and other factors was alleviated by Bacillus licheniformis supplementation. *PLoS One,* 2017, vol. 12
- **XU.** Bacillus licheniformis normalize the ileum microbiota of chickens infected with necrotic enteritis. *Sci. Rep.,* 2018, vol. 8, 1744
- **WADE, B. ; KEYBURN, A.L.** The true cost of necrotic enteritis. *World Poultry,* 2015, vol. 31, 16-17
- **ROOD, J. I.** Virulence genes of Clostridium perfringens. *Annual Review of Microbiology,* 1998, vol. 52, 333-360
- **POPOFF, M. R. ; P. BOUVET.** Genetic characteristics of toxigenic Clostridia and toxin gene evolution. *Toxicon,* 2013, vol. 75, 63-89
- **KEYBURN, A. L. et al.** Alpha-toxin of Clostridium perfringens is not an essential virulence factor in necrotic enteritis in chickens. *Infection and Immunity,* 2006, vol. 74 (11), 6496-6500

- **KEYBURN, A. L. et al.** NetB, a new toxin that is associated with avian necrotic enteritis caused by Clostridium perfringens. *PLoS Pathogens,* 2008, vol. 4, 2
- **KALDHUSDAL ; LØVLAND.** The economical impact of Clostridium perfringens is greater than anticipated. *World Poultry,* 2000, vol. 16, 50-51
- **SKINNER et al.** An economic analysis of the impact of subclinical (mild) necrotic enteritis in broiler chickens. *Avian Dis.,* 2010, vol. 54 (4), 1237-1240
- **M'SADEQ et al.** Towards the control of necrotic enteritis in broiler chickens with in-feed antibiotics phasing-out Worldwide. *Animal Nutrition,* 2015, vol. 1, 11
- **DAHIYA et al.** MD. Potential strategies for controlling necrotic enteritis in broiler chickens in post-antibiotic era. *Anim. Feed Sci. Technol.,* 2006, vol. 129, 60
- **CRESSMAN.** Interrelations between the microbiotas in the litter and in the intestines of commercial broiler chickens. *Appl. Environ. Microbiol.,* 2010, vol. 76 (19), 6572-82
- **BORDA-MONILA.** Current Perspectives of the Chicken Gastrointestinal Tract and Its Microbiome. *Computational and Structural Biotechnology Journal,* 2018, vol. 16, 131-139
- **DANZEISEN.** Modulations of the chicken cecal microbiome and metagenome in response to anticoccidial and growth promoter treatment. *PLoS ONE,* 2011, vol. 6, 11
- **YILMAZ.** Uniting the classification of cultured and uncultured bacteria and archaea using 16S rRNA gene sequences. *Nat. Rev. Microbiol.,* 2014, vol. 12 (9), 635-45
- **STANLEY.** Highly variable microbiota development in the chicken gastrointestinal tract. *PLoS ONE,* 2013, vol. 8, 12
- **SERGEANT.** Extensive microbial and functional diversity within the chicken cecal microbiome. *PLoS ONE,* 2014, vol. 14, 9-3
- **BORDA-MOLINA.** Insights into broilers' gut microbiota fed with phosphorus, calcium and phytase supplemented diets. *Front Microbiol,* 2016, vol. 7